# EUROPÄISCHE PATENTSCHRIFT

(11) **EP 1 518 522 B1**
(45) Veröffentlichungstag und Bekanntmachung des Hinweises auf die Patenterteilung: **02.01.2008**
(21) Anmeldenummer: 04090499.7
(22) Anmeldetag: 15.02.2001
(51) Int. Cl.: A61F 5/01, A61F 5/02, A61F 5/37

(54) **Vorrichtung zum Halten eines Körpergelenks**
Apparatus for maintaining a body articulation
Dispositif pour maintenir une articulation corporelle

(30) Priorität: 15.02.2000 DE 20003254 U
(43) Veröffentlichungstag der Anmeldung: 30.03.2005
(62) Teilanmeldung aus: 01250050.0
(73) Patentinhaber: Lob, Tobias, 81377 München (DE)
(72) Erfinder: Lob, Tobias, 81377 München (DE)
(74) Vertreter: Eisenführ, Speiser & Partner

(56) Entgegenhaltungen:
- GB-A- 2 290 032
- US-A- 3 528 413
- US-A- 5 312 323
- US-A- 5 514 081
- US-A- 5 645 079

## Beschreibung

Die Erfindung betrifft eine Vorrichtung zum Halten eines Körpergelenks, insbesondere eines Knies, in einer definierten Lage gemäß dem Oberbegriff des Anspruchs 1.

Zur Diagnose von Verletzungen des Bandapparates im Bereich von Körpergelenken sind zahlreiche Hilfsmittel bekannt. So ist beispielsweise zur Diagnose von Bänderrissen im Bereich des Sprunggelenks eine Vorrichtung bekannt, bei der Ferse und Unterschenkel jeweils in einem Halteelement fixiert werden. Anschließend wird über einen im Bereich des Sprunggelenks auf den Unterschenkel einwirkenden Vorschubmechanismus ein definierter Knickwinkel des Sprunggelenks eingestellt, um dann in dieser Lage unter anderem Röntgenaufnahmen von der betroffenen Stelle anfertigen zu können und so Rückschluß auf Art und Ausmaß der Verletzung zu erlangen.

Diese Vorrichtungen weisen jedoch den Nachteil auf, daß sie durch die Vorgabemöglichkeit lediglich des Knick- bzw. Beugewinkels des betreffenden Körpergelenks nur begrenzte Diagnosemöglichkeiten zur Verfügung stellen. Durch die Vorgabe der Knickung bzw. Beugung des Gelenks ist es lediglich möglich, die Verletzung insoweit zu erfassen, als die in dieser Stellung voneinander entfernten Rißenden des Bandes beispielsweise ertastbar oder auf einem Röntgenbild erkennbar sind. Weitergehende Diagnosemöglichkeiten eröffnen diese Vorrichtungen ebensowenig wie die Möglichkeit, in der Nachsorge zu Rückschlüssen über den Heilungsprozeß zu gelangen.

Weiterhin sind Vorrichtungen der eingangs genannten Art bekannt, die unter anderem zur Ruhigstellung des Körpergelenks in einer bestimmten Winkellage verwendet werden. Auch sind Beinorthesen bekannt, bei denen das Kniegelenk nach der Behandlung einer Verletzung des Bandapparats etc. in einer bestimmten Beuge- bzw. Streckstellung fixiert wird, um zur Wiederherstellung der normalen Beweglichkeit einen Dehnungsreiz auf verkürzte Bänder oder Weichteile auszuüben und diese somit wieder zu verlängern. In beiden Fällen sind Halteelemente zum Fixieren der durch das Körpergelenk verbundenen Körperteile vorgesehen, die zum Einstellen eines definierten Beugewinkels des Körpergelenks arretierbar gelenkig miteinander verbunden sind. So ist z.B. aus der US 5,645,079 eine Vorrichtung bekannt, die dazu dient, einen Körperteil eines Patienten während einer orthopädischen Untersuchung in einer bestimmten Position, mit vorgegebenes Beugewinkel, Drehwinkel und Streckungsgrad zu fixieren, wobei die Vorrichtung fest mit einem Bettgestell verbunden ist, um die Position des besagten Körperteils während der Untersuchung vollständig festzulegen.

Die genannten Vorrichtungen werden dabei zum einen in der Regel nicht zur Diagnose der jeweiligen Verletzung verwendet. Zum anderen würden auch sie, für den Fall, daß sie hierfür eingesetzt würden, wie die oben genannten Diagnoseeinrichtungen den Nachteil nur sehr begrenzter Diagnosemöglichkeiten aufweisen.

Der Erfindung liegt deshalb die Aufgabe zugrunde, eine Vorrichtung der eingangs genannten Art zur Verfügung zu stellen, welche einen größere Bandbreite an Diagnosemöglichkeiten bietet.

Die Aufgabe wird, ausgehend von einer Vorrichtung gemäß dem Oberbegriff des Anspruchs 1, durch die im kennzeichnenden Teil des Anspruchs 1 angegebenen Merkmale gelöst.

Die Erfindung schließt die technische Lehre ein, daß man eine Erweiterung der Diagnosemöglichkeiten mit den gattungsgemäßen Vorrichtungen erzielt, wenn wenigstens eine mit wenigstens einem Halteelement zusammenwirkende erste Einrichtung zum Aufbringen einer definierten Kraft auf das Körpergelenk vorgesehen ist.

Neben den bereits bekannten Diagnosemöglichkeiten, die auch mit der erfindungsgemäßen Vorrichtung uneingeschränkt gegeben sind, kann zum einen anhand der durch die Krafteinwirkung induzierten Bewegung im Gelenk auf Art und Ausmaß der Verletzung geschlossen werden.

Zum anderen ist die Vorrichtung auch in vorteilhafter Weise in der Nachsorge einzusetzen. So ist es mit der erfindungsgemäßen Vorrichtung bei Nachsorgeuntersuchungen möglich, erneut dieselbe definierte Kraft auf das Gelenk aufzubringen und anhand der Änderung der durch die Einwirkung induzierten Bewegung im Gelenk Rückschlüsse auf den Heilungsprozeß zu ziehen. Alternativ kann auch die Kraft erfaßt werden, die zum Erzielen einer bestimmten Bewegung erforderlich ist, und aus der Änderung der Kraft gegenüber vormaligen Messungen der Heilungsprozeß beurteilt werden. Aus dem Stand der Technik, wie z.B. der US 5,514,081 sind zwar Vorrichtungen bekannt, welche eine definierte Kraft auf ein Gelenk ausüben, aber auch diese Vorrichtungen eignen sich nur unzureichend für die Durchführung von Untersuchungen, da es hierbei oft notwendig ist, dass das Gelenk erstens einen bestimmten Beugegrad aufweist und darüber hinaus auch noch lokal fixiert ist.

Bei der erfindungsgemäßen Vorrichtung ist eine mit wenigstens einem der Halteelemente verbundene dritte Einrichtung zum Befestigen an einer Lagereinrichtung, insbesondere einer Liege oder einem Stuhl, vorgesehen. Hierdurch ist es möglich, die betroffenen Körperteile und damit in Grenzen auch den Patienten in einer bestimmten Position an dieser Lagereinrichtung, beispielsweise einer Liege, zu fixieren und somit zu verhindern, daß er den zu untersuchenden Körperbereich während der Untersuchung unbeabsichtigt bewegen kann. Dies ist besonders bei Aufnahmen im Kernspintomographen oder dergleichen von Vorteil.

Vorzugsweise umfaßt die erste Einrichtung wenigstens ein Kraftausübungsmittel zum Aufprägen der Kraft auf das Körpergelenk. Dieses kann in beliebiger bekannter Weise ausgebildet sein. So ist es z. B. möglich, eine oder mehrere entsprechend vorspannbare Federn einzusetzen, die dann je nach Vorspannung eine definierte Kraft auf das Körpergelenk aufprägen.

Bei besonders günstigen Varianten der erfindungsgemäßen Vorrichtung ist das Kraftausübungsmittel zum Aufbringen der Kraft über hydrostatischen Druck, vorzugsweise über den hydrostatischen Druck eines Gases, insbesondere Luft, ausgebildet, da sich die Vorrichtung hierbei besonders einfach aufbauen läßt und sich die ausgeübte Kraft in einfacher Weise durch die Variation des hydrostatischen Druckes verändern läßt. Die ausgeübte Kraft läßt sich dabei einfach anhand eines entsprechenden Druckmessers umgehend ermitteln.

Besonders günstig, insbesondere im Hinblick auf die Herstellungskosten, läßt sich dies bei Ausführungen realisieren, bei denen das Kraftausübungsmittel als aufpumpbares Kissen ausgebildet ist.

Die Vorrichtung kann so ausgebildet sein, daß eine Kraft aus unterschiedlichen Richtungen auf das Gelenk aufgeprägt werden kann. Hierzu kann das Kraftausübungsmittel beispielsweise in seiner Lage zu den Halteelementen veränderlich befestigbar sein. Es können aber auch mehrere Kraftausübungsmittel an unterschiedlichen Stellen vorgesehen sein, mit denen dann eine in Betrag und Richtung variierbare resultierende Kraft erzielt werden kann.

Die Kraft kann unmittelbar in das Gelenk eingeleitet werden. Bevorzugt ist das Kraftausübungsmittel aber derart ausgebildet und angeordnet, daß die Kraft auf einen der über das Körpergelenk verbundenen Körperteile ausgeübt wird, da sich hierbei aufgrund der in der Regel günstigeren geometrischen Verhältnisse in den vom Gelenk etwas entfernter liegenden Bereichen eine einfachere und universeller einzusetzende Vorrichtung ergibt.

Besonders günstige Varianten der erfindungsgemäßen Vorrichtung zeichnen sich dadurch aus, daß die erste Einrichtung wenigstens ein Übertragungselement zum Übertragen der Kraft des Kraftausübungsmittels auf den Körperteil umfaßt. Hierdurch reduziert sich der für die Ausgestaltung und Herstellung des Kraftausübungsmittels erforderliche Aufwand, da es keine unmittelbare Auflagefläche für den betreffenden Körperteil aufweisen muß.

Eine Änderung der definierten Kraft, die auf das Gelenk aufgeprägt wird, kann durch Austauschen der Kraftausübungsmittel erfolgen. Bevorzugte Ausführungen der erfindungsgemäßen Vorrichtung besitzen eine erste Einrichtung mit Stellmitteln zum Einstellen der Kraft. Dank ihres einfachen Aufbaus besonders günstige Ausführungen heben sich dadurch hervor, daß die Krafterzeugung über hydrostatischen Druck erfolgt und die Stellmittel eine Pumpe und ein Ablaßventil umfassen. Die Kraft läßt sich hierdurch besonders schnell und einfach variieren.

Bevorzugt sind erfindungsgemäß ausgebildete Vorrichtungen für Untersuchungen eines Knie- oder eines Schultergelenks vorgesehen.

Bei weiteren bevorzugten Varianten der erfindungsgemäßen Vorrichtung ist zusätzlich wenigstens eine mit wenigstens einem Halteelement zusammenwirkende zweite Einrichtung zum Einstellen eines definierten Verdrehwinkels des Körpergelenks vorgesehen. Hierdurch sind die Diagnosemöglichkeiten der Vorrichtung noch zusätzlich erweitert, da hierbei nun auch der Einfluß der Verdrehung der Körperteile zueinander berücksichtigt werden kann.

Im Bereich des Knies kann dies beispielsweise durch eine entsprechende zum Halteelement des Unterschenkels um dessen Längsachse in unterschiedlichen Drehstellungen arretierbare Fußfixierung erfolgen.

Das Vorsehen wenigstens einer mit wenigstens einem Halteelement zusammenwirkenden zweiten Einrichtung zum Einstellen eines definierten Verdrehwinkels des Körpergelenks stellt im übrigen, ausgehend von den eingangs genannten gattungsbildenden Vorrichtungen, auch schon für sich einen eigenständigen Erfindungsgedanken dar, der die obengenannte Aufgabe löst, die Diagnosemöglichkeiten mit der Vorrichtung auszubauen.

Günstige Weiterbildungen der erfindungsgemäßen Vorrichtung zeichnen sich dadurch aus, daß sie im wesentlichen aus einem bzw. mehreren Materialien bestehen, die sich im wesentlichen nicht störend auf Magnetfelder auswirken. Hierdurch sind störungsfreie Aufnahmen der betroffenen Körperstelle in der fixierten Lage beispielsweise im Kernspintomographen möglich.

Vorzugsweise besteht die Vorrichtung dabei im wesentlichen aus einem oder mehreren Kunststoffen. Hierfür kommen aufgrund ihrer günstigen mechanischen Eigenschaften insbesondere Polyethylen (PE), Polyamid (PA) und Polyoxymethylen (POM) in Betracht.

Die Arretierung der Halteelemente in der jeweils vorgegebenen Beugestellung kann durch eine entsprechend verriegelbare Ausgestaltung der Gelenkverbindung zwischen den Halteelementen gegeben sein. Sie kann auch durch eine oder mehrere entsprechende Streben zwischen den Halteelementen realisiert sein. Es ist jedoch auch möglich, daß die dritte Einrichtung zum Arretieren der Halteelemente zum Einstellen des definierten Beugewinkels des Körpergelenks ausgebildet ist.

Andere vorteilhafte Weiterbildungen der Erfindung sind in den Unteransprüchen gekennzeichnet bzw. werden nachstehend zusammen mit der Beschreibung der bevorzugten Ausführungen der Erfindung anhand der Figuren näher dargestellt. Es zeigen:
- Figur 1: einen Schnitt durch ein bevorzugtes Ausführungsbeispiel der Erfindung,
- Figur 2: eine Seitenansicht eines weiteren bevorzugten Ausführungsbeispiels der Erfindung,
- Figur 3: ein weiteres Ausführungsbeispiel der Erfindung in Ansicht von vorn, sowie
- Figur 4: das in Figur 3 gezeigte Ausführungsbeispiel der Erfindung in Ansicht von oben.

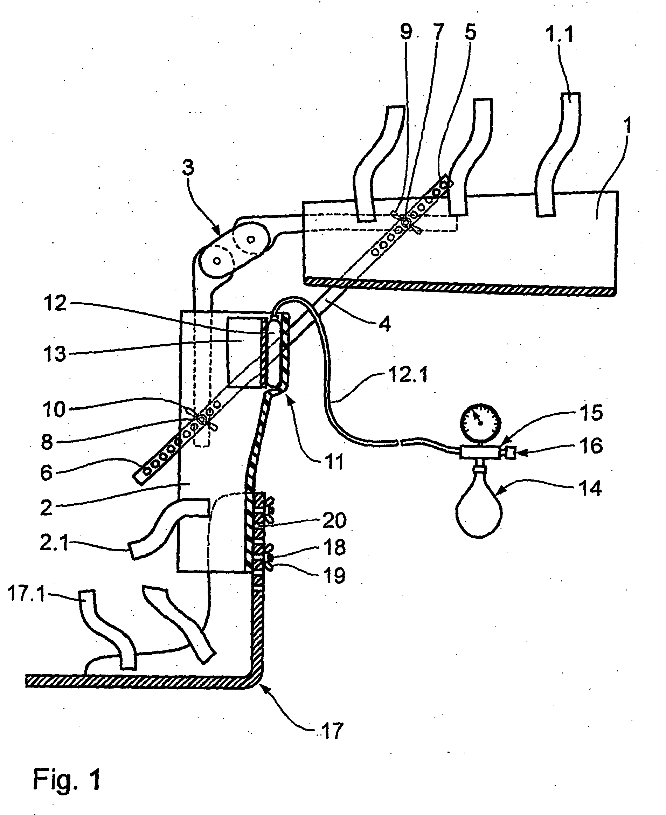

Figur 1 zeigt einen Schnitt durch ein Ausführungsbeispiel einer erfindungsgemäßen Vorrichtung zum Halten eines Körpergelenks, hier eines Knies, in einer definierten Lage. Die Vorrichtung weist eine als erstes Halteelement dienende Oberschenkelschale 1 zur Fixierung des Oberschenkels und eine als zweites Halteelement dienende Unterschenkelschale 2 zur Fixierung des Unterschenkels auf. Die Fixierung des jeweiligen Körperteils erfolgt dabei über Klettbänder 1.1 bzw. 2.1. Die Oberschenkelschale 1 und die Unterschenkelschale 2 sind über zwei Doppelgelenke 3 gelenkig miteinander verbunden, die im ans Bein angelegten Zustand medial und lateral des Knies angeordnet sind.

Zum Einstellen und Arretieren eines definierten Beugewinkels des Knies ist jeweils medial und lateral eine Strebe 4 vorgesehen, welche die Oberschenkelschale 1 und die Unterschenkelschale 2 miteinander verbindet. Die Strebe 4 ist an ihren Enden jeweils mit einer Anzahl von Bohrungen 5 bzw. 6 versehen. Durch diese Bohrungen 5 bzw. 6 kann sie auf an der Oberschenkelschale 1 bzw. der Unterschenkelschale 2 angeordnete Befestigungsstifte 7 bzw. 8 aufgesteckt werden. Die Befestigungsstifte 7 bzw. 8 sind als Gewindestifte ausgebildet, so daß die Strebe 4 in dieser Lage durch Flügelmuttern 9 bzw. 10 fixiert werden kann. Je nach Abstand der beiden mit den Befestigungsstiften 7 und 8 in Eingriff befindlichen Bohrungen 5 bzw. 6 ergibt sich ein bestimmter Beugewinkel des Kniegelenks.

Es versteht sich hierbei, daß anstelle der einzelnen Bohrungen in der Strebe auch zumindest an einem Ende der Strebe ein Langloch vorgesehen sein kann, um die Winkelverstellbarkeit zu gewährleisten. Ebenso versteht sich, daß bei anderen Varianten der Erfindung zum einen auch eine einzige Strebe genügen kann und zum anderen die Arretierung auch ohne Streben durch ein in unterschiedlichen Positionen verriegelbares Gelenk zwischen der Ober- und Unterschenkelschale realisiert sein kann.

Am gelenkseitigen Ende der Unterschenkelschale 2 ist eine erste Einrichtung 11 zum Aufbringen einer definierten Kraft auf das Kniegelenk vorgesehen. Diese erste Einrichtung 11 umfaßt innen in der Unterschenkelschale 2 in einer Ausbuchtung der Schale 2 angeordnete Kraftausübungsmittel 12. Die Kraft wird pneumatisch erzeugt. Die Kraftausübungsmittel sind hierzu von einem aufpumpbaren Kissen 12 gebildet.

Das Kissen 12 stützt sich auf der einen Seite gegen die Unterschenkelschale 2 und auf der anderen Seite gegen eine Wadenschale 13 ab, die als Anlage für die Wade des Patienten und somit als Übertragungsmittel zum Übertragen der Kraft des Kissens 12 auf den Unterschenkel dient. Die Kraft wird somit nicht direkt, sondern über die Wade in das Gelenk eingeleitet. Damit sich eine möglichst gute Krafteinleitung in das Kniegelenk ergibt und durch diese Krafteinleitung induzierte Bewegungen im Kniegelenk nicht behindert werden, wird der Unterschenkel des Patienten nur am dem Gelenk abgewandten Ende an der Unterschenkelschale 2 über das Klettband 2.1 fixiert.

Das Aufpumpen des Kissens 12 erfolgt über einen Schlauch 12.1 durch einen Luftdruckbeutel 14, der mit einer Manometerpumpe 15 versehen ist. Hiermit kann der im Kissen vorherrschende hydrostatische Druck und damit die durch das Kissen auf den Unterschenkel ausgeübte Kraft eingestellt werden. Zum Ablassen der Luft und damit zum Reduzieren des Druckes bzw. der ausgeübten Kraft dient ein Ablaßventil 16. Luftdruckbeutel 14, Manometerpumpe 15 und Ablaßventil 16 fungieren somit als Stellmittel zum Einstellen der Kraft auf das Kniegelenk.

Je nach eingestelltem Druck, d. h. je nach eingestellter Kraft im Kniegelenk, ergeben sich in diesem Reaktionen, insbesondere Verlagerungen des Gelenks, die einen Rückschluß auf die Art und das Ausmaß der Verletzung erlauben. Ebenso kann im Zuge der Nachsorge durch den Vergleich der Gelenkreaktionen bei jeweils denselben Drücken im Kissen 12 und damit bei denselben ins Kniegelenk eingeleiteten Kräften auf den Heilungsfortschritt geschlossen werden.

Am dem Gelenk 3 abgewandten Ende der Unterschenkelschale 2 ist eine zweite Einrichtung 17 zum Einstellen eines definierten Verdrehwinkels des Kniegelenks angeordnet. Die zweite Einrichtung besteht dabei aus einer Fußschale 17, an welcher der Fuß des Patienten durch Klettbänder 17.1 fixiert werden kann.

Die Fußschale ist an der Unterschenkelschale 2 über zwei Flügelmuttern 19 befestigt, die auf zwei an der Unterschenkelschale 2 angeordneten Gewindestiften 18 aufgeschraubt sind. Die Gewindestifte 18 sind dabei in Langlöchern 20 in der Fußschale 17 angeordnet, die im wesentlichen in einer Ebene senkrecht zur Längsachse der Unterschenkelschale 2 verlaufen.

Die Langlöcher 20 ermöglichen es, je nach ihrer Bogenlänge, die Fußschale 17 um einen bestimmten Winkel relativ zur Unterschenkelschale 2 um deren Längsachse zu verdrehen und in beliebigen Verdrehpositionen über die Flügelmuttern 19 festzuklemmen. Durch die Verdrehung des in der Fußschale 17 fixierten Fußes wird auch der in der Unterschenkelschale 2 fixierte Unterschenkel des Patienten in entsprechender Weise verdreht und somit auch ein definierter Verdrehwinkel im Kniegelenk eingestellt.

Hierdurch ist es möglich, Verletzungen, die bei normaler, d. h. unverdrehter Fußhaltung bzw. unverdrehtem Gelenk nicht oder schwer zu erkennen sind, zuverlässig und in einfacher Weise zu diagnostizieren, ohne hierfür die Hilfe einer zusätzlichen Person zu benötigen, welche den Fuß des Patienten in der Verdrehstellung halten müßte.

Um den Abstand der Fußschale 17 vom Gelenk 3 an den jeweiligen Patienten anpassen zu können, sind eine Anzahl von Langlöchern 20 in Längsrichtung der Unterschenkelschale 2 aneinandergereiht.

Es versteht sich im übrigen, daß bei anderen Varianten der erfindungsgemäßen Vorrichtung zur Verstellung des Verdrehwinkels der Fußschale anstelle der Langlöcher auch einzelne entsprechend verteilte Bohrungen vorgesehen sein können, wobei dann der Verdrehwinkel natürlich nur noch in diskreten Schritten variiert werden kann.

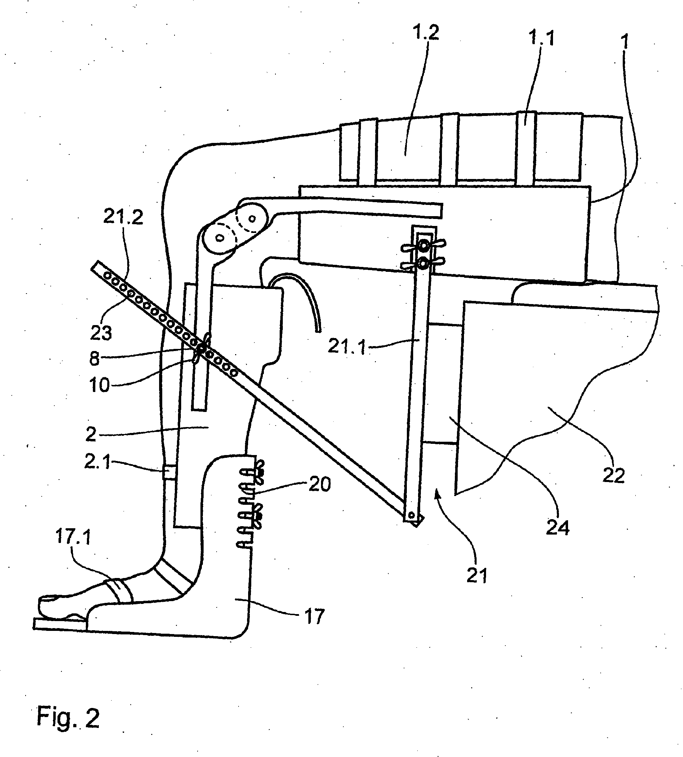

Figur 2 zeigt eine Seitenansicht einer weiteren Ausführung der erfindungsgemäßen Vorrichtung mit einem Bein, das mit Hilfe der Klettbänder 1.1, 2.1 und 17.1 in der Oberschenkelschale 1, der Unterschenkelschale 2 und der Fußschale 17 fixiert ist, wobei auf den Oberschenkel noch eine zweite Oberschenkelschale 1.2 aufgelegt ist. Diese Ausführung entspricht im wesentlichen derjenigen aus Figur 1, weshalb hier lediglich auf die Unterschiede eingegangen werden soll.

Der Unterschied besteht darin, daß eine mit der Oberschenkelschale 1 und der Unterschenkelschale 2 verbundene dritte Einrichtung 21 vorgesehen ist, über welche die Vorrichtung an einer Liege 22 oder dergleichen befestigt werden kann. Die dritte Einrichtung umfaßt medial und lateral des Beines jeweils einen ersten Arm 21.1, der fest mit der Oberschenkelschale 1 verbunden ist, und einem zweiten Arm 21.2, der mit der Unterschenkelschale 2 verbunden ist. An ihrem der jeweiligen Schale 1 bzw. 2 abgewandten Ende sind die Arme 21.1 und 21.2 gelenkig miteinander verbunden.

Die Befestigung des zweiten Arms 21.2 an der Unterschenkelschale 2 erfolgt über den Gewindestift 8 und die Flügelmutter 10. Die dritte Einrichtung 21 übernimmt im gezeigten Beispiel die Funktion des Arretierens der Ober- und Unterschenkelschale 1 und 2 in einer bestimmten Winkelstellung. Um diese Winkelstellung und damit den Beugewinkel des Kniegelenks einstellen zu können, weist der zweite Arm 21.2 in seiner Längsrichtung eine Reihe von Bohrungen 23 auf, durch die der Gewindestift 8 geführt werden kann.

Die Befestigung an der Liege 22 erfolgt dabei über ein Flanschelement 24, an dem die ersten Arme 21.1 befestigt sind. Hierdurch ergibt sich eine Fixierung der Vorrichtung relativ zur Liege, wodurch der Patient gehindert ist, das Bein unbeabsichtigt zu bewegen. Dies ist für die Untersuchung, insbesondere aber für Aufnahmen im Kernspintomographen oder dergleichen von großem Vorteil.

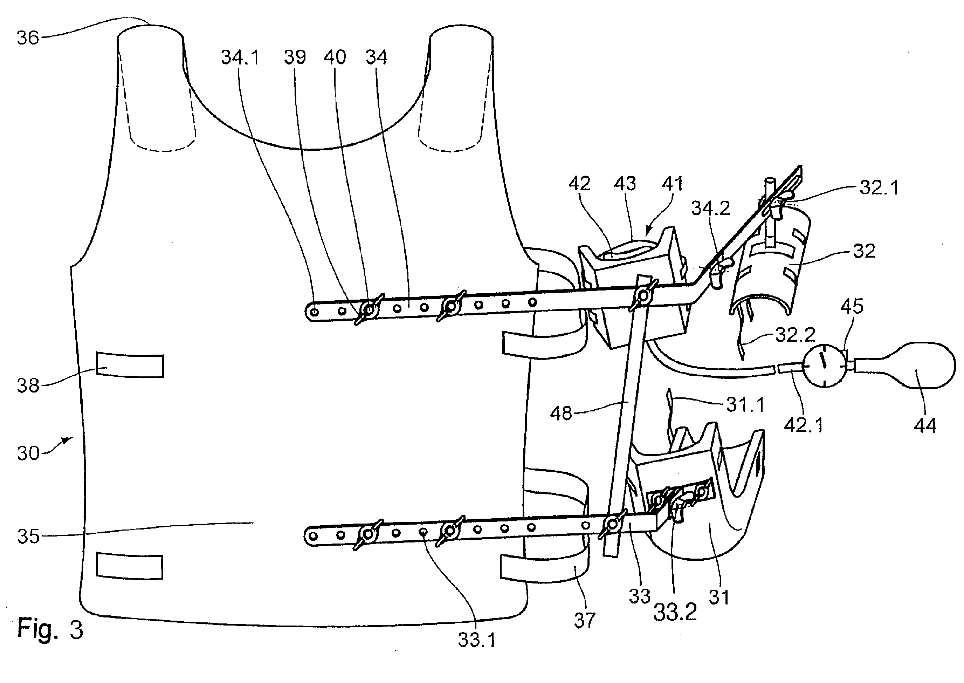

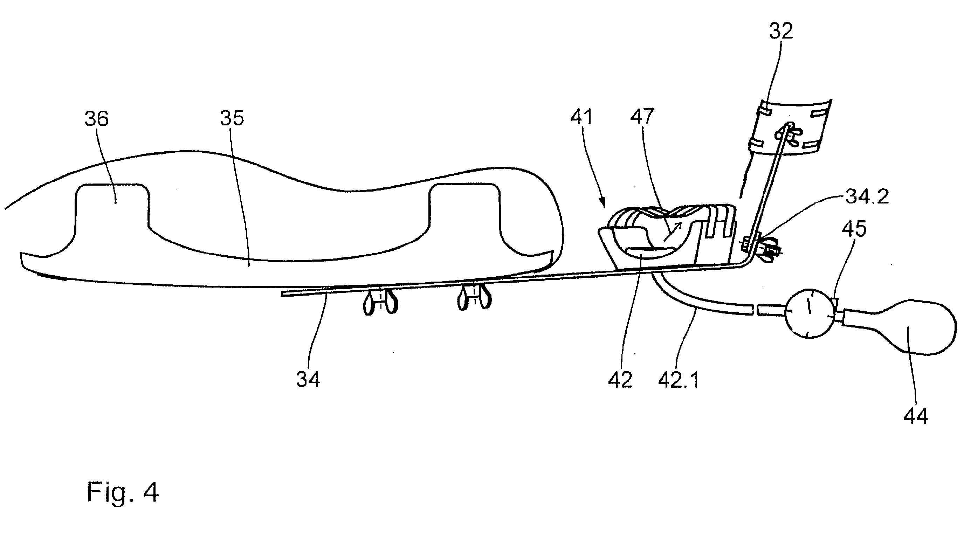

In den Figuren 3 und 4 ist in einer perspektivischen Seitenansicht und in Draufsicht ein weiteres Ausführungsbeispiel einer erfindungsgemäßen Vorrichtung 30 zum Untersuchen von Schulterluxationen und refixierten Schultergelenken gezeigt, mit weicher das Handgelenk und das Ellenbogengenlenk bezüglich des Schultergelenks in einer definierten Lage gehalten werden können.

Die Vorrichtung 30 weist eine als erstes Halteelement dienende Ellenbogenschale 31 zur Fixierung des Ellenbogengelenks und eine als zweites Halteelement dienende Handgelenkschale 32 zur Fixierung des Unterarms in einer vorgebbaren Position auf. Die Fixierung des jeweiligen Körperteils an den Schalen 31 und 32 erfolgt dabei über bevorzugt als Klettbänder ausgebildete Schnellverschlüsse 31.1 bzw. 32.2. Die Ellenbogenschale 31 und die Handgelenkschale 32 sind jeweils über eine Haltestrebe 33, 34 mit einem am Rücken eines Patienten zu positionierende Korsett 35 verbunden. Das Korsett 35 weist zwei Schulterbügel 36 und Klettverschlußmittel 37 und 38 auf, mittels denen es am Körper des Patienten fixiert werden kann.

In den Haltestreben 33, 34 sind zwecks Anpassung an unterschiedliche Körpermaße jeweils mehrere, in Reihe angeordnete Befestigungslöcher 33.1, 34.1 vorgesehen, mittels derer die Streben unter Verwendung von Flügelmuttern 39 an in der Korsettwandung vorgesehene Bolzen 40 angeschraubt werden. Es versteht sich, daß das Korsett 35 selbst auch größenverstellbar ausgebildet sein kann, um auch hier eine Anpassung an die unterschiedlichsten Körpermaße vornehmen zu können. Zum Einstellen und Arretieren eines definierten Beugewinkels des Ellenbogengelenks ist die Handgelenkschale 32 gelenkig an dem freien Ende der Haltestrebe 34 verbunden. Das freie Ende der Haltestrebe 34 ist selbst schwenkbar ausgebildet. Die gelenkigen Verbindungsstellen an den Streben sind mit 33.2 bzw. 34.2 bezeichnet.

An der Haltestrebe 34 ist gleichzeitig eine Einrichtung 41 zum Aufbringen einer definierten Kraft auf das Schultergelenk vorgesehen, welche als Oberarmschale ausgebildet den Oberarm dorsal umgreift. Die Einrichtung 41 umfaßt ein innenseitig an der Schale angeordnetes Kraftausübungsmittel 42. Die Kraft wird pneumatisch erzeugt. Das Kraftausübungsmittel ist hierzu als aufpumpbares Kissen 42 gebildet.

Das Kissen 42 stützt sich auf der einen Seite gegen die Oberarmschale 41 ab, die als Anlage für den Oberarm des Patienten und somit als Übertragungsmittel zum Übertragen der Kraft des Kissens 12 auf den Oberarm dient. Die Kraft wird somit nicht direkt, sondern über den Oberarm in das Schultergelenk eingeleitet. Damit sich eine möglichst gute Krafteinleitung in das Schultergelenk ergibt und durch diese Krafteinleitung induzierte Bewegungen im Schultergelenk nicht behindert werden, wird der Unterarm des Patienten nur am dem Gelenk abgewandten Ende an der Ellenbogenschale 31 über das Klettband 31.1 fixiert.

Das Aufpumpen des Kissens 42 erfolgt über einen Schlauch 42.1 durch einen Luftdruckbeutel 43, der mit einer Manometerpumpe 44 versehen ist. Hiermit kann der im Kissen vorherrschende hydrostatische Druck und damit die durch das Kissen auf den Oberarm ausgeübte Kraft eingestellt werden. Zum Ablassen der Luft und damit zum Reduzieren des Druckes bzw. der ausgeübten Kraft dient ein Ablaßventil 45. Luftdruckbeutel 43, Manometerpumpe 44 und Ablaßventil 45 fungieren somit als Stellmittel zum Einstellen der Kraft auf das Schultergelenk. Die Richtung der Kraft ist durch den Pfeil 47 angedeutet.

Je nach eingestelltem Druck, d. h. je nach eingestellter Kraft im Schultergelenk, ergeben sich in diesem Reaktionen, insbesondere Verlagerungen des Gelenks, die einen Rückschluß auf die Art und das Ausmaß der Verletzung erlauben. Ebenso kann im Zuge der Nachsorge durch den Vergleich der Gelenkreaktionen bei jeweils denselben Druckwerten im Kissen 42 und damit bei denselben ins Schultergelenk eingeleiteten Kräften auf den Heilungsfortschritt geschlossen werden.

Zur Sicherung einer ausreichenden mechanischen Stabililtät weist die Vorrichtung 30 eine zusätzliche Strebe 48 auf, welche die Haltestreben 33 und 34 lateral verbindet.

Die Erfindung beschränkt sich in ihrer Ausführung nicht auf die vorstehend angegebenen bevorzugten Ausführungsbeispiele. Vielmehr ist eine Anzahl von Varianten im Rahmen der beanspruchten Vorrichtung möglich.

## Patentansprüche

1. Vorrichtung zum Halten eines Körpergelenks in einer definierten Lage mit Halteelementen (1, 2, 31, 32) zum Fixieren der durch das Körpergelenk verbundenen Körperteile, die zum Einstellen eines definierten Beugewinkels des Körpergelenks arretierbar gelenkig miteinander verbunden sind, wobei wenigstens eine mit wenigstens einem Halteelement (2, 32) zusammenwirkende erste Einrichtung (11, 41) zum Aufbringen einer definierten Kraft auf das Körpergelenk vorgesehen ist, **dadurch gekennzeichnet, daß** eine mit wenigstens einem der Halteelemente (1, 2) verbundene dritte Einrichtung (21, 33, 34) zum Befestigen an einer Lagereinrichtung (22, 35), insbesondere einer Liege oder einem Stuhl, vorgesehen ist.

2. Vorrichtung nach Anspruch 1, **dadurch gekennzeichnet, daß** die erste Einrichtung (11, 41) wenigstens ein Kraftausübungsmittel (12, 42) zum Aufprägen der Kraft auf das Körpergelenk umfaßt.

3. Vorrichtung nach Anspruch 2, **dadurch gekennzeichnet, daß** das Kraftausübungsmittel (12, 42) zum Aufbringen der Kraft über hydrostatischen Druck ausgebildet ist.

4. Vorrichtung nach Anspruch 3, **dadurch gekennzeichnet, daß** das Kraftausübungsmittel (12, 42) zum Aufbringen der Kraft über hydrostatischen Druck eines Gases, insbesondere Luft, ausgebildet ist.

5. Vorrichtung nach Anspruch 3 oder 4, **dadurch gekennzeichnet, daß** das Kraftausübungsmittel als aufpumpbares Kissen (12, 42) ausgebildet ist.

6. Vorrichtung nach einem der Ansprüche 2 bis 5, **dadurch gekennzeichnet, daß** das Kraftausübungsmittel (12, 42) derart ausgebildet und angeordnet ist, daß die Kraft auf einen der über das Körpergelenk verbundenen Körperteile ausgeübt wird.

7. Vorrichtung nach einem der Ansprüche 2 bis 6, **dadurch gekennzeichnet, daß** die erste Einrichtung (11, 41) wenigstens ein Übertragungselement (13, 43) zum Übertragen der Kraft des Kraftausübungsmittels (12, 42) auf den Körperteil umfaßt.

8. Vorrichtung nach einem der vorhergehenden Ansprüche, **dadurch gekennzeichnet, daß** die erste Einrichtung (11, 41) Stellmittel (14, 15, 16, 44, 45, 46) zum Einstellen der Kraft umfaßt.

9. Vorrichtung nach Anspruch 8, **dadurch gekennzeichnet, daß** die Krafterzeugung über hydrostatischen Druck erfolgt und die Stellmittel eine Pumpe (15, 45) und ein Ablaßventil (16, 46) umfassen.

10. Vorrichtung nach einem der vorhergehenden Ansprüche, **dadurch gekennzeichnet, daß** wenigstens eine mit wenigstens einem Halteelement (2) zusammenwirkende zweite Einrichtung (17) zum Einstellen eines definierten Verdrehwinkels des Körpergelenks vorgesehen ist.

11. Vorrichtung nach einem der vorhergehenden Ansprüche, **dadurch gekennzeichnet, daß** sie im wesentlichen aus einem bzw. mehreren Materialien besteht, die sich im wesentlichen nicht störend auf Magnetfelder auswirken.

12. Vorrichtung nach Anspruch 11, **dadurch gekennzeichnet, daß** sie im wesentlichen aus einem oder mehreren Kunststoffen besteht.

13. Vorrichtung nach Anspruch 1, **dadurch gekennzeichnet, daß** die dritte Einrichtung (21) zusätzlich zur Befestigung der Halteelemente an der Lagereinrichtung zum Einstellen des definierten Beugewinkels des Körpergelenks zum Arretieren der Halteelemente (1, 2) ausgebildet ist.

## Claims

1. Device fot holding a body joint in a defined position with holding elements (1, 2, 31, 32) for fixing the body parts connected by the body joint which are connected together in a securable, articulating manner to set a defined bending angle of the body joint, wherein at least one first device (11, 41) interacting with at least one holding element (2, 32) is provided for applying a defined force onto the body joint, **characterised in that** a third device (21, 33, 34) connected with at least one of the holding elements (1, 2) is provided for securing onto a bearing device (22, 35), in particular a couch or a chair.

2. Device according to claim 1, **characterised in that** the first device (11, 41) comprises at least one force exerting means (12, 42) for impressing the force onto the body joint.

3. Device according to claim 2, **characterised in that** the force exerting means (12, 42) is designed for applying force by means of hydrostatic pressure.

4. Device according to claim 3, **characterised in that** the force exerting means (12, 42) is designed for applying force by means of the hydrostatic pressure of a gas, in particular air.

5. Device according to claim 3 or 4, **characterised in that** the force exerting means is designed as an inflatable cushion (12,42).

6. Device according to one of claims 2 to 5, **characterised in that** the force exerting means (12, 42) is designed and arranged so that force is exerted onto one of the body parts connected by the body joint.

7. Device according to one of claims 2 to 6, **characterised in that** the first device (11, 41) comprises at least one transfer element (13, 43) for transferring the force of the force exerting means (12, 42) onto the body part.

8. Device according to one of the preceding claims, **characterised in that** the first device (11, 41) comprises adjusting means (14, 15, 16, 44, 45, 46) for adjusting the force.

9. Device according to claim 8, **characterised in that** force is generated by means of hydrostatic force and the adjusting means comprises a pump (15, 45) and an outlet valve (16, 46).

10. Device according to one of the preceding claims, **characterised in that** at least one second device (17) cooperating with at least one holding element (2) is provided for setting a defined rotational angle of the body joint.

11. Device according to one of the preceding claims, **characterised in that** it is made essentially from one or more materials which do not have a negative effect on magnetic fields.

12. Device according to claim 11, **characterised in that** it is made essentially from one or more plastics.

13. Device according to claim 1, **characterised in that** the third device (21) is designed in addition to securing the holding elements on the bearing device for adjusting the defined bending angle of the body joint to lock the holding elements (1, 2).

## Revendications

1. Dispositif destiné à maintenir une articulation corporelle dans une position définie, comprenant des éléments de retenue (1, 2, 31, 32) qui sont destinés à bloquer les parties du corps assemblées par l'articulation corporelle et qui sont assemblés entre eux de manière articulée immobilisable pour régler un angle de pliage défini de l'articulation corporelle, au moins un premier appareillage (11, 41) coopérant avec au moins un élément de retenue (2, 32) étant prévu pour appliquer une force définie sur l'articulation corporelle, **caractérisé en ce qu'**il est prévu un troisième appareillage (21, 33, 34) qui est relié à au moins un des éléments de retenue (1, 2) et qui est destiné à être fixé contre un dispositif de repos (22, 35), en particulier une surface de couchage ou une chaise.

2. Dispositif selon la revendication 1, **caractérisé en ce que** le premier appareillage (11, 41) comporte au moins un moyen d'application de force (12, 42) pour appliquer la force sur l'articulation corporelle.

3. Dispositif selon la revendication 2, **caractérisé en ce que** le moyen d'application de force (12, 42) est conçu pour appliquer la force par l'intermédiaire d'une pression hydrostatique.

4. Dispositif selon la revendication 3, **caractérisé en ce que** le moyen d'application de force (12, 42) est conçu pour appliquer la force par l'intermédiaire d'une pression hydrostatique d'un gaz, en particulier l'air.

5. Dispositif selon la revendication 3 ou 4, **caractérisé en ce que** le moyen d'application de force est conçu sous forme de coussin gonflable (12, 42).

6. Dispositif selon l'une quelconque des revendications 2 à 5, **caractérisé en ce que** le moyen d'application de force (12, 42) est conçu et est agencé de telle sorte que la force est exercée sur l'une des parties du corps assemblées par l'intermédiaire d'une articulation corporelle.

7. Dispositif selon l'une quelconque des revendications 2 à 6, **caractérisé en ce que** le premier appareillage (11, 41) comporte au moins un élément de transmission (13, 43) destiné à transmettre la force du moyen d'application de force (12, 42) sur la partie du corps.

8. Dispositif selon l'une quelconque des revendications précédentes, **caractérisé en ce que** le premier appareillage (11, 41) comporte des moyens de réglage (14, 15, 16, 44, 45, 46) destinés à régler la force.

9. Dispositif selon la revendication 8, **caractérisé en ce que** la force est générée par l'intermédiaire de la pression hydrostatique et les moyens de réglage comportent une pompe (15, 45) et une vanne d'évacuation (16, 46).

10. Dispositif selon l'une quelconque des revendications précédentes, **caractérisé en ce qu'**au moins un deuxième appareillage (17) coopérant avec au moins un élément de retenue (2) est prévu pour régler un angle de torsion défini de l'articulation corporelle.

11. Dispositif selon l'une quelconque des revendications précédentes, **caractérisé en ce que** ledit dispositif est réalisé sensiblement en un ou plusieurs matériaux qui n'influent sensiblement pas de manière gênante sur les champs magnétiques.

12. Dispositif selon la revendication 11, **caractérisé en ce que** ledit dispositif est réalisé sensiblement en une ou plusieurs matières plastiques.

13. Dispositif selon la revendication 1, **caractérisé en ce que** le troisième appareillage (21), en plus d'être destiné à fixer les éléments de retenue contre le dispositif de repos, est conçu pour régler l'angle de pliage défini de l'articulation corporelle, pour bloquer les éléments de retenue (1, 2).
